# DEMANDE DE BREVET EUROPEEN

(11) **EP 3 409 386 A1**
(43) Date de publication de la demande: **05.12.2018**
(21) Numéro de dépôt: 18175339.3
(22) Date de dépôt: 31.05.2018
(51) Int. Cl.: B08B 15/04, F04F 5/22, F04F 5/46

(54) **DISPOSITIF D'ASPIRATION DE FUMEES CHIRURGICALES EN BLOC OPERATOIRE**

(30) Priorité: 31.05.2017 FR 1754809
(71) Demandeur: Apar, 77220 Tournan-en-Brie (FR)
(72) Inventeur: LOPES, José, Joao, 77330 OZOIR-LA-FERRIERE (FR)
(74) Mandataire: Cabinet Beau de Loménie

(57) **Abrégé**

Dispositif d'aspiration de fumées chirurgicales comportant :
- un premier orifice d'entrée (100) pour recevoir un tuyau souple d'aspiration de fumées relié à un instrument de chirurgie générant des fumées chirurgicales tel un bistouri électrique,
- un module de filtration (16, 110) pour traiter les fumées chirurgicales admises par le premier orifice d'entrée (100) et en extraire un air purifié, et
- un générateur de vide à effet venturi comportant au moins une chambre de détente annulaire (114A, 114B, 114C) recevant l'air purifié issu du module de filtration et permettant à partir d'air comprimé reçu par un second orifice d'entrée (102) de créer une dépression dans au moins une chambre de détente annulaire (114A, 114B, 114C), l'air purifié étant mélangé à l'air comprimé avant d'être refoulés ensemble par un orifice de sortie (104).

L'adjonction d'une électrovanne commandée (20) en amont du générateur de vide à effet venturi permet en outre un gain énergétique en consommation d'air comprimé.

## Description

La présente invention concerne un dispositif d'aspiration en bloc opératoire permettant d'aspirer les fumées chirurgicales créées lors d'opérations de chirurgie notamment au moyen de bistouris électriques.

L'utilisation de bistouris électriques en bloc opératoire est maintenant courante et tend à remplacer le bistouri traditionnel dans les interventions nécessitant la découpe de tissu humain ou animal. Toutefois, lors de cette utilisation, l'évaporation ou la vaporisation du liquide cellulaire (constitué à 95% d'eau et de 5% de débris) est source de fumées dont la nocivité est maintenant reconnue (odeurs, irritations pulmonaires ou oculaires, nausées dues à la présence de substances chimiques, etc...) et qu'il convient donc impérativement d'évacuer rapidement du champ opératoire pour assurer la protection des personnels exposés et limiter la possible contamination de l'air des blocs opératoires.

Il est connu de recourir aux dispositifs muraux d'aspiration de liquide ou de gaz d'anesthésie déjà présents en bloc opératoire pour évacuer ces fumées. Toutefois, cette solution ne peut être envisagée que de façon ponctuelle et n'est pas satisfaisante en cas d'utilisation récurrente.

Aussi, il a été proposé avec la demande de brevet CH 672883, un dispositif mobile d'évacuation et de traitement de fumées comportant une pompe à air actionnée par un moteur. Ce dispositif n'est malheureusement pas dépourvu d'inconvénients notamment du fait de la nécessaire compatibilité électromagnétique d'un tel dispositif et du recours à un moteur nécessairement bruyant et dès lors peu conciliable avec l'ambiance feutrée et calme du bloc opératoire notamment lors d'anesthésies sous hypnose aujourd'hui de plus en plus fréquentes.

### Objet et résumé de l'invention

L'objet de la présente invention est donc de proposer un dispositif d'aspiration de fumées chirurgicales en bloc opératoire qui pallie les inconvénients précités, soit de structure simple et en outre adapté à tout type de bloc opératoire. Un autre objet est de proposer un dispositif économe en énergie, notamment quant à la consommation d'air pneumatique (médical ou technique).

Ce but est atteint par un dispositif d'aspiration de fumées chirurgicales comportant :
- un premier orifice d'entrée pour recevoir un tuyau souple d'aspiration de fumées,
- un module de filtration pour traiter les fumées chirurgicales admises par le premier orifice d'entrée et en extraire un air purifié, caractérisé en ce qu'il comporte en outre :
- un générateur de vide à effet venturi comportant au moins une chambre de détente annulaire recevant l'air purifié issu du module de filtration et permettant à partir d'air comprimé reçu par un second orifice d'entrée de créer une dépression dans ladite au moins une chambre de détente annulaire, l'air purifié étant mélangé à l'air comprimé avant d'être refoulés ensemble par un orifice de sortie et en ce que ledit tuyau souple d'aspiration de fumées est relié à un instrument de chirurgie générant des fumées chirurgicales, tel un bistouri électrique.

Le dispositif ainsi obtenu ne nécessite pas d'alimentation électrique et s'avère donc autonome. Sans aucune pièce en mouvement, il permet de réduire les nuissances sonores et en recourant au seul circuit d'air comprimé de la salle d'opération, il limite les proliférations microbiennes résultant de l'utilisation de l'air ambiant comme générateur d'aspiration.

Avantageusement, le module de filtration comporte un filtre à particules précédé d'un filtre à charbon actif.

De préférence, le générateur de vide à effet venturi comporte au moins une buse au travers de laquelle s'écoule l'air comprimé et au moins une chambre de détente annulaire entourant ladite au moins une buse et recevant par un orifice latéral d'aspiration l'air purifié issu directement du module de filtration, la dépression créée dans la chambre de détente annulaire par l'accélération de la vitesse de l'air comprimé traversant la buse aspirant l'air purifié et le mélangeant à l'air comprimé préalablement à leur refoulement par l'orifice de sortie.

Avantageusement, ladite au moins une chambre de détente annulaire débouche dans une chambre annulaire de répartition comportant l'orifice latéral d'aspiration recevant l'air purifié issu directement du module de filtration.

De préférence, le générateur de vide à effet venturi comporte trois buses montées en ligne et au travers de laquelle s'écoule successivement l'air comprimé et trois chambres de détente annulaires entourant chacune des trois buses et recevant par un orifice latéral d'aspiration l'air purifié issu directement du module de filtration, la dépression créée dans les chambres de détente annulaires successives par l'accélération de la vitesse de l'air comprimé traversant successivement les trois buses aspirant l'air purifié et le mélangeant à l'air comprimé préalablement à leur refoulement par l'orifice de sortie.

Avantageusement, les trois chambres de détente annulaires comportent chacune une entrée circulaire ouvrant dans une même chambre annulaire de répartition comportant l'orifice latéral d'aspiration recevant l'air purifié issu directement du module de filtration.

Un silencieux est avantageusement monté sur l'orifice de sortie.

Avantageusement, le filtre à particules est un filtre à fibres de verre à très haute efficacité ou très faible pénétration. Il est de préférence amovible.

De préférence, l'air comprimé est issu d'un réseau d'air pneumatique présent dans tout bloc opératoire et le tuyau souple d'aspiration est fixé directement sur un conduit d'évacuation faisant partie intégrante de l'instrument de chirurgie ou d'un ensemble de maintien de cet instrument de chirurgie.

Avantageusement, une électrovanne commandée est disposée avant le générateur de vide à effet venturi au niveau du second orifice d'entrée, cette électrovanne étant actionnée via un circuit électronique associé à partir d'un signal de commande reçu d'une pince ampèremétrique disposée autour d'un fil d'alimentation électrique de l'instrument de chirurgie.

### Brève description des dessins

D'autres caractéristiques et avantages de la présente invention ressortiront de la description qui va maintenant être poursuivie en relation avec les figures annexées sur lesquelles :
- la figure 1 est une vue en perspective montrant un instrument chirurgical couplé à un dispositif d'aspiration de fumées chirurgicales selon l'invention,
- la figure 2 illustre schématiquement le dispositif d'aspiration de fumées chirurgicales de la figure 1, et
- la figure 3 détaille un exemple de module de génération de vide à effet venturi.

### Description détaillée d'un mode de réalisation

Le principe à la base de l'invention repose sur l'utilisation d'un générateur de vide à effet venturi pour créer à partir d'air comprimé un vide propre à aspirer les fumées chirurgicales issues du champ opératoire. Ainsi, aucune source électrique n'est nécessaire pour créer la dépression assurant cette aspiration.

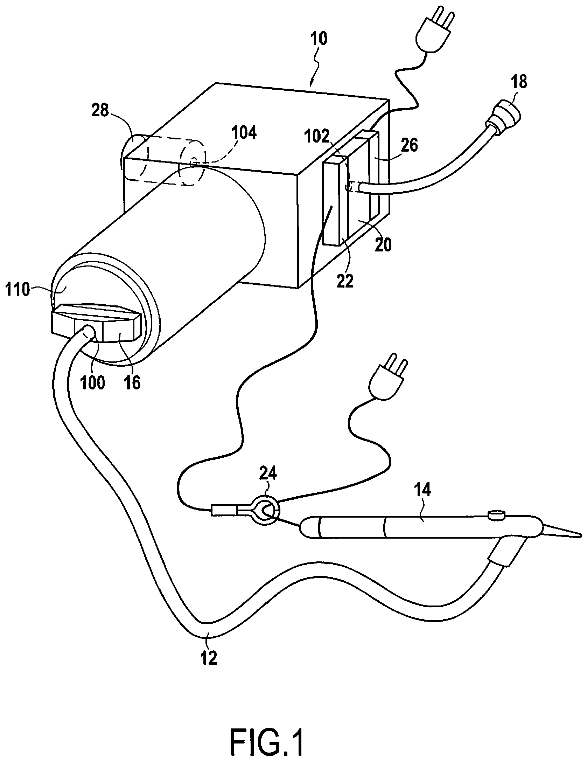

La figure 1 illustre en perspective un dispositif d'aspiration de fumées chirurgicales 10 conforme à l'invention et tel qu'il se présente en bloc opératoire avec un tuyau souple d'aspiration 12 pour sa liaison à un instrument de chirurgie 14, par exemple un bistouri électrique, à ultrasons ou à laser. Cette connexion au niveau du bistouri peut être effectuée au niveau d'un conduit d'évacuation qui peut faire partie intégrante de l'instrument ou d'un ensemble de maintien de cet instrument et sur lequel le tuyau souple d'aspiration est alors fixé.

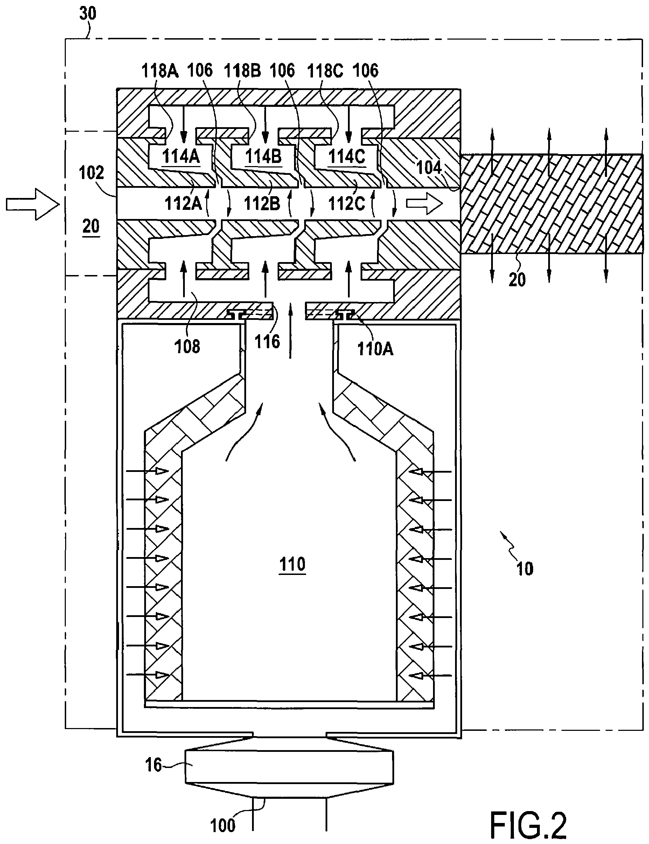

Le dispositif d'aspiration de fumées chirurgicales 10 dont la structure interne sera décrit plus en détail en regard de la figure 2, est relié, par un premier orifice d'entrée 100, au tuyau souple 12 d'aspiration des fumées chirurgicales et par un second orifice d'entrée 102, au réseau d'air pneumatique 18 (air médical ou air technique comprimé à disposition dans tous les blocs opératoires). De préférence, un filtre à charbon actif 16 est disposé sur le trajet du tuyau souple d'aspiration 12 en aval de l'instrument de chirurgie 14 pour filtrer les mauvaises odeurs dégagées lors de l'opération et aspirées au travers de ce tuyau souple d'aspiration. Ainsi, lors de la phase d'aspiration des fumées qui s'effectue en continu, les fumées passent tout d'abord par ce filtre à charbon actif pour être débarrassées de ces odeurs avant d'être purifiées des particules et autres microorganismes qu'elles transportent dans un filtre à particules 110, de préférence amovible.

Afin d'optimiser le fonctionnement du dispositif d'aspiration de fumées chirurgicales et notamment sa consommation d'air pneumatique, une électrovanne commandée 20 peut-être montée sur l'alimentation en air du dispositif au niveau du second orifice d'entrée 102, avant le générateur de vide à effet venturi, pour permettre ou non cette arrivée d'air et donc corrélativement activer ou non le processus d'aspiration des fumées chirurgicales. L'électrovanne est commandée par un circuit électronique 22 contenant une loi de commande déterminée (en pratique une carte électronique à microcontrôleur) permettant d'actionner l'ouverture ou la fermeture de cette électrovanne en fonction d'un signal de commande reçu d'une pince ampèremétrique 24 disposée autour du fil d'alimentation électrique alimentant l'instrument de chirurgie 14. Une alimentation électrique 26 de préférence munie d'un transformateur, par exemple 24 V (tension non invasive pour un patient), assure l'alimentation en énergie de l'électrovanne et de son circuit électronique de commande ainsi que de la pince ampèremétrique.

En fonctionnement, le tuyau souple d'aspiration des fumées générées par le bistouri électrique doit tout d'abord être raccordé au dispositif d'aspiration qui est lui-même connecté au réseau d'air comprimé, de préférence via l'électrovanne 20. Un orifice de sortie 104 muni d'un ensemble réducteur de bruit 28 de type silencieux assure le retour de l'air purifié dans le bloc opératoire. Le silencieux peut être de type diffusant ou débouchant, le premier étant toutefois privilégié du fait de sa meilleure atténuation sonore au risque toutefois d'un éventuel colmatage impossible dans le second. L'ensemble de ces composants est avantageusement disposé dans une mallette portative 30 (voir la figure 2) à laquelle sera reliée l'alimentation en air et éventuellement le câblage électrique (non illustré) lié à l'adjonction de l'électrovanne.

Le processus d'aspiration des fumées peut alors débuter lorsque qu'un courant électrique est détecté par la pince ampèremétrique 24 positionnée autour du fil d'alimentation électrique de l'instrument de chirurgie 14, la commande de l'électrovanne 20 permettant d'actionner cette aspiration en autorisant l'arrivée d'air depuis le réseau 18. Cette commande reste active tant que l'instrument de chirurgie est lui-même actif. Lorsque l'instrument de chirurgie n'est plus actif, le processus d'aspiration est stoppé automatiquement après un nombre déterminé de secondes, paramétrable au niveau du circuit électronique 22, suivant l'arrêt de la détection du courant électrique.

La figure 2 illustre plus particulièrement la structure interne du dispositif d'aspiration de fumées chirurgicales 10 organisé autour d'un module de filtration et d'un générateur de vide à effet venturi permettant d'assurer un débit d'air important couplé à une forte dépression. Ce générateur de vide reçoit par l'orifice d'entrée 102, de préférence via l'électrovanne 20, de l'air médical ou de l'air comprimé (par exemple à 6 bars de pression) du réseau pneumatique 18 et refoule cet air par l'orifice de sortie 104 muni du silencieux 28. L'effet de ou des restrictions formant venturi 106 dans le passage de cet air comprimé permet en aspirant l'air extrait du champ opératoire et pénétrant dans le dispositif par le premier orifice d'entrée 100 de créer un vide (de l'ordre de 0,9 bars) dans une chambre d'aspiration 108 à laquelle ce premier orifice est relié via un filtre à particules 110 délivrant un air purifié. Le filtre à particules avantageusement amovible via une liaison démontable 110A (à vis ou baïonnette par exemple) pour en faciliter le remplacement (consommable périodique), est par exemple un filtre à très haute efficacité de type HEPA (high efficiency particulate air) ou très faible pénétration de type ULPA (ultra low pénétration air) permettant de capturer de très fines particules (inférieures à 0,3 ou 0,1 micron) et des microorganismes, bactéries ou virus. Un modèle adéquat est un filtre en fibres de verre de classe H13 ou H14 par exemple.

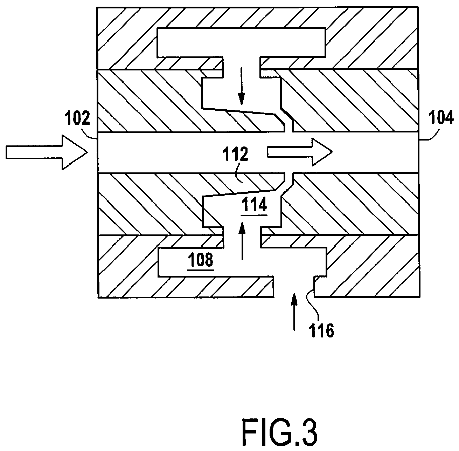

La figure 3 montre un exemple basique de générateur de vide à effet Venturi mise en oeuvre dans le dispositif de l'invention. Il repose sur l'utilisation d'une buse 112 au travers de laquelle l'air comprimé issu du réseau 18 et introduit par l'orifice d'entrée 102 s'écoule et d'une chambre de détente annulaire 114 entourant cette buse et recevant par un orifice latéral d'aspiration 116 l'air purifié, c'est-à-dire celui extrait du champ opératoire une fois débarrassé de ses mauvaises odeurs (par le filtre à charbon actif 18) et expurgé de ses particules fines (par le filtre à particules 110). La dépression créée dans la chambre de détente annulaire par l'accélération de la vitesse de l'air comprimé traversant la buse va alors par effet du vide créé aspirer l'air purifié se présentant à l'orifice d'entrée de cette chambre annulaire, lequel air purifié va se mélanger avec l'air comprimé et être refoulé ensemble vers l'orifice de sortie 104 avant de traverser le silencieux 20 au travers duquel ils vont diffuser ensuite dans le bloc opératoire.

Revenons à la figure 2 et plus particulièrement au générateur de vide à effet Venturi qui présente, contrairement à la structure de la figure 3 à chambre de détente annulaire unique 112, une structure multi-chambres permettant d'obtenir un fort débit aspiré. On notera que cette structure permet de créer un effet venturi grâce à plusieurs chambres et cela avec une seule alimentation d'air comprimé.

Plus particulièrement, dans la structure illustrée, trois buses 112A, 112B, 112C entourées chacune par une chambre de détente annulaire 114A, 114B, 114C sont montées en ligne, l'une derrière l'autre, de façon à former trois étages de dépression successifs, chaque chambre de détente annulaire comportant une entrée circulaire 118A, 118B, 118C ouvrant de préférence dans la chambre d'aspiration 108 formant une même chambre de répartition annulaire et comportant l'orifice latéral d'aspiration 116. Avec cette configuration, la dépression engendrée par le premier étage dans la première chambre annulaire 114A va générer un débit d'air aspiré qui va se mélanger à l'air comprimé pour former un premier mélange d'air. Ce premier mélange d'air va passer dans le deuxième étage et créer une nouvelle dépression dans la deuxième chambre annulaire 114B générant un nouveau débit d'air aspiré qui ajouté au premier mélange d'air va former un deuxième mélange. Ce deuxième mélange va enfin passer dans le troisième étage et créer une dernière dépression dans la troisième chambre annulaire 114C de sorte à générer un troisième débit d'air aspiré qui mélangé avec le deuxième mélange va délivrer en sortie de la structure un mélange d'air final refoulé au travers du silencieux diffusant.

L'invention ainsi décrite procure notamment les avantages suivants :
- il s'agit d'un système entièrement statique avec aucun mouvement mécanique et donc sans utilisation d'une pompe à vide,
- il est de faible poids et peu encombrant et peut donc tenir dans une simple mallette portative de quelques kilos,
- l'aspiration ne nécessite aucune alimentation électrique (sauf en cas d'adjonction de l'électrovanne commandée) et demande seulement une connexion au réseau d'air pneumatique,
- l'aspiration des fumées est obtenue à partir du réseau d'air pneumatique et non de l'air ambiant du bloc opératoire. L'air expulsé avec les fumées traitées est donc un air qui peut être qualifié de «propre»,
- le générateur de vide est avantageusement monté dans un caisson insonorisé supprimant tout risque de bruit,
- le débit d'aspiration est optimisé grâce à l'utilisation de plusieurs chambres de détente,
- l'adjonction de l'électrovanne commandée permet un gain énergétique en consommation d'air pneumatique puisque le dispositif d'aspiration ne peut être activé que lorsque l'instrument de chirurgie est utilisé ainsi qu'un confort sonore accru du fait d'une durée utilisation réduite.

## Revendications

1. Dispositif d'aspiration de fumées chirurgicales (10) comportant :
- un premier orifice d'entrée (100) pour recevoir un tuyau souple d'aspiration de fumées (12),
- un module de filtration (16, 110) pour traiter les fumées chirurgicales admises par le premier orifice d'entrée (100) et en extraire un air purifié, **caractérisé en ce qu'**il comporte en outre :
- un générateur de vide à effet venturi comportant au moins une chambre de détente annulaire (114 ; 114A, 114B, 114C) recevant l'air purifié issu du module de filtration et permettant à partir d'air comprimé reçu par un second orifice d'entrée (102) de créer une dépression dans ladite au moins une chambre de détente annulaire, l'air purifié étant mélangé à l'air comprimé avant d'être refoulés ensemble par un orifice de sortie (104), et **en ce que** ledit tuyau souple d'aspiration de fumées est relié à un instrument de chirurgie (14) générant des fumées chirurgicales, tel un bistouri électrique.

2. Dispositif selon la revendication 1, **caractérisé en ce que** le module de filtration est amovible et comporte un filtre à particules (110) précédé d'un filtre à charbon actif (16).

3. Dispositif selon la revendication 2, **caractérisé en ce que** le filtre à particules (110) est un filtre à fibres de verre à très haute efficacité ou très faible pénétration.

4. Dispositif selon la revendication 1 ou la revendication 2, **caractérisé en ce que** le générateur de vide à effet venturi comporte au moins une buse (112) au travers de laquelle s'écoule l'air comprimé et au moins une chambre de détente annulaire (114) entourant ladite au moins une buse et recevant par un orifice latéral d'aspiration (116) l'air purifié issu directement du module de filtration, la dépression créée dans la chambre de détente annulaire par l'accélération de la vitesse de l'air comprimé traversant la buse aspirant l'air purifié et le mélangeant à l'air comprimé préalablement à leur refoulement par l'orifice de sortie (104).

5. Dispositif selon la revendication 4, **caractérisé en ce que** ladite au moins une chambre de détente annulaire débouche dans une chambre annulaire de répartition (108) comportant l'orifice latéral d'aspiration (116) recevant l'air purifié issu directement du module de filtration.

6. Dispositif selon la revendication 1 ou la revendication 2, **caractérisé en ce que** le générateur de vide à effet venturi comporte trois buses (112A, 112B, 112C) montées en ligne et au travers de laquelle s'écoule successivement l'air comprimé et trois chambres de détente annulaires (114A, 114B, 114C) entourant chacune des trois buses et recevant par un orifice latéral d'aspiration (116) l'air purifié issu directement du module de filtration, la dépression créée dans les chambres de détente annulaires successives par l'accélération de la vitesse de l'air comprimé traversant successivement les trois buses aspirant l'air purifié et le mélangeant à l'air comprimé préalablement à leur refoulement par l'orifice de sortie (104).

7. Dispositif selon la revendication 6, **caractérisé en ce que** les trois chambres de détente annulaires comportent chacune une entrée circulaire (118A, 118B, 118C) ouvrant dans une même chambre annulaire de répartition (108) comportant l'orifice latéral d'aspiration (116) recevant l'air purifié issu directement du module de filtration.

8. Dispositif selon l'une quelconque des revendications 1 à 7, **caractérisé en ce qu'**il comporte en outre un silencieux (20) monté sur l'orifice de sortie (104).

9. Dispositif selon l'une quelconque des revendications 1 à 8, **caractérisé en ce que** l'air comprimé est issu d'un réseau d'air pneumatique (18) présent dans tout bloc opératoire.

10. Dispositif selon l'une quelconque des revendications 1 à 9, **caractérisé en ce que** le tuyau souple d'aspiration (12) est fixé directement sur un conduit d'évacuation faisant partie intégrante de l'instrument de chirurgie (14) ou d'un ensemble de maintien de cet instrument de chirurgie.

11. Dispositif selon l'une quelconque des revendications 1 à 10, **caractérisé en ce qu'**il comporte en outre une électrovanne commandée (20) disposée avant le générateur de vide à effet venturi au niveau du second orifice d'entrée (102).

12. Dispositif selon la revendication 11, **caractérisé en ce que** ladite électrovanne est actionnée via un circuit électronique associé (22) à partir d'un signal de commande reçu d'une pince ampèremétrique (24) disposée autour d'un fil d'alimentation électrique de l'instrument de chirurgie.
